# EUROPEAN PATENT SPECIFICATION

(11) **EP 3 225 155 B1**
(45) Date of publication and mention of the grant of the patent: **29.04.2020**
(21) Application number: 08425006.7
(22) Date of filing: 04.01.2008
(51) Int. Cl.: A61B 5/00, A61B 5/107

(54) **Spinal meter**
Wirbelsäulenmesser
Appareil de mesure du rachis

(30) Priority: 12.01.2007 IT TR20070001
(43) Date of publication of application: 04.10.2017
(73) Proprietor: Banyan Technologies GmbH, 9500 Villach (AT)
(72) Inventor: Bellavigna, Gianluca, 05100 Terni (IT)
(74) Representative: Romano, Giuseppe

(56) References cited:
- WO-A2-2005/082249
- DRERUP B ET AL: "Back shape measurement using video rasterstereography and three-dimensional reconstruction of spinal shape", CLINICAL BIOMECHANICS, BUTTERWORTH SCIENTIFIC LTD, GUILDFORD, GB, vol. 9, no. 1, 1 January 1994 (1994-01-01) , pages 28-36, XP026409323, ISSN: 0268-0033 [retrieved on 1994-01-01]

## Description

We developed the project taking into consideration two main points of view: technical innovation and care to the costumer (patient in this case). The moving cause of our project was the possibility to realize a new device such to allow findings on the spine without absorption of radiations. Presently, a correct spine's exam can only be carried out trough radiography. Today the radiographic method is the most common and exact one also because there are no alternatives but visual conventional exams.

On a side, the radiography is today the best method to diagnose a scoliosis or a secondary paramorphism, on the other side this method has considerable consequences on health and creates serious ethical-diagnostic considerations to the operators.

The absorption of any radiation represents an any case a risk, specially in the teenage, with consequent worry for parents.

Always under the ethical-diagnostic point of view it's to be underlined too the difficulties for specialists, orthopedists, physiotherapists or dentists, in exposing the patient to several radiographies to diagnose a pathology or establish improvements or aggravations in relation with the medical control or with the treatments put into effect to normalize the pathology.

Thanks to Spinal Meter now it's possible to obtain all the advantages of radiography without its disadvantages. Also Spinal Meter allows the doctor to realize assessments that should be impossible with standard radiography. On example of a back shape measurement system for three dimensional reconstitution and assessment of the spline without radiography is discloses in the Article "Back shape measurement using video rasterstereography and three-dimensional reconstruction of spinal shape" published in CLINICAL BIOMECHANICS, BUTTERWORTH SCIENTIFIC LTD, GUILDFORD, GB, vol. 9, no. 1, 1 January 1994 (1994-01-01), pages 28-36.

From the point o view of patient's health, it's possible to repeat medicals at close range: the absence of radiations represents a big chance and can be a good incentive to undergo preventive screenings.

The ethical-diagnostic point of view gets a decisive transformation. The doctor can put into execution exams on the spine to young and adult patients and will follow the clinical iter in the best way, without worries.

As far as diagnosis is concerned, Spinal Meter will allow medicals to examine, for example, the curvature of the spine, to put immediately into operation the corrective (for example an orthodontic byte or an arch support) doing after few minutes another medical to check the result of the corrective.

We can add that the doctor, with Spinal Meter, will be able to point out automatically the length of **a portion of** upper and lower limbs and avoid long calculation that are manual so far (Cobb's angle).

Practically a revolution.

### 2. GENERAL DESCRIPTION

This document describes the actions and what is necessary to build a prototype of "Spinal Meter". The invention is a device as defined by claim 1.

The project concerns the application of this technological innovation in medical and paramedical area.

Spinal Meter allows an important modification of the techniques used for diagnosis and monitoring on patients with vertebral pathology, secondary paramorphism and articular dysmetry.

The use of this device has important consequences on public as well as on private field. Namely it allows a drastic costs reduction connected with the radiographic exam, a better control of patients personal data and also a consistent time-saving.

Fundamental elements in the project phase have been the study of the specific anatomic points on which to apply the luminous TAG and the setting up of the specialist software able to produce from the image and from the graphic TAG, the specific diagrams, data and parameters of the dorsal curve and the patient's limbs.

### 3. COMPONENTS

Spinal Meter is the result of several components connected each other and no divisible. Only the interaction among the components allows, as final result, the Spinal Meter exam.

The components are:
1) Personal Computer;
2) Image acquisition and coaxial illumination System;
3) Position's reflectors applied on patient;
4) Elaboration and acquisition data software.
5) Platform

### 3.1. Personal Computer

Personal Computer with these basic characteristics needs:
CPU PIII 1 GHz or superior;
Operating system Windows XP;
Ram 2 Gb;
Fixed disk with at least 1 Gbyte free;
Monitor with at least 1024X768 Pixel resolution;
Door USB for the images acquisition's system.

### 3.2 Peripherals (image acquisition and coaxial illumination)

This system can be composed both by a Web Cam and by an acquisition video's source WDM compatible (image's acquisition card connected to a video camera) and also by a digital camera CANON POWER-SHOT.

With the first two solutions the system gets a streaming video from the sources above-said: when the operator decides to get the image, the system memorizes the photogram then present on the stream video.

With the third possibility the software guides the acquisition of a single photogram by the digital camera.

The system is completed by the coaxial light that allows to distinguish the position reflectors placed on the patient anatomic points. The illuminator can consist of the camera's flash or video light.

### 3.3 Position reflectors and anatomic indexing points

The position reflectors (TAG) are formed by a reflecting band of second class and have hypoallergenic adhesive to stick on anatomic points of the patient. The points we located to allow Spinal Meter to execute the survey are:
Cervical Iliac crest right
Lumbar Iliac crest left
Thoracic Axillary Plica left
Sacral Axillary Plica right
Scapula left Popliteal left
Scapula right Popliteal right
Elbow left Heel left
Elbow right
Heel right

### 3.4 Software of elaboration and acquisition data

This program allows, trough the automatic research of the position of the reflectors on the patient's image, to create the back's curve and analyze it for verifying eventual anomalies. In this software there are algorithm of video elaboration, of positions research from an image, of fit of a diagram and of analyzed data storage.

### 3.5 Platform

The platform is equipped with incorporate level and anatomic support.

### 4. APPLICATION'S AREAS

Orthopedics
Dentistry
Physiotherapy
Osteopathy
Odontostomatology
Chiroterapy
Khinesitherapy
Posturolugy
Remedial Gymnastic
Postural Gymnastic
Fisiokinnesiterapy

### 5. ADVANTAGES

The use of Spinal Meter allows to:
An efficiency improvement consisting in:
Drastic reduction of costs connected with a radiological check up;
Increase of medical performances;
Optimization of patient's personal data handling;
Saving time, that means removal of the wait necessary to run a radiography;
Possibility to repeat tests in the same session without radiations absorption;
Possibility to put into prompt effect correctives and to verify the result (Byte; Plantar);
A Spinal Meter exam is practically free of charge;
Improve the service quality for the patient trough:
   Reduction or total elimination of radiation absorption;
   Saving time because the traditional check up needs a previous radiological visit and a second specialist one. With Spinal Meter there will be only one invention.

### 6. DATA OF THE PROJECT

A portable device must be available.

This apparatus allows the user to visualize in a very simple way, after the setting of luminous Tag and the acquisition of the image on the Personal computer, the images in 3D worked out by the software and the data concerning the spine, the scapulas and upper and lower limbs.

The exam also allows to store the patient's information and the medical results (images and data) and to record all the information useful for the future.

Another important functional character of Spinal Meter is the possibility to calculate Cobb's Angle, in automatic or manual mode.

It is also possible to insert in Spinal Meter radiographies in digital format and make comparisons and monitoring with Spinal Meter results.

The patient will get the medical record in papery or in digital format (CD ROM). In this way the patient that moves to another place, can address to different specialist (using Spinal Meter), with all the information of his previous clinical pictures.

### 7. DESCRIPTION OF THE PROJECT of Spinal Meter components

Thanks to small-size of Spinal Meter components, they can be set in a small suitcase. The operations needed to obtain the Spinal Meter test are very simple:
luminous TAG setting;
connection, trough the USB door of the peripheral (Camera o Web Cam) to the Personal Computer;
acquisition of images or photograms;
elaboration;
print and/or copy on hardware support;

The apparatus, described in these pages has, among its components, the software of acquisition and elaboration data, that carries out a very important role in the employment of the all diagnostic system. In fact, thanks to the use of this program is possible to reproduce in 3D the spine's curve of the patient, trough the automatic research of the reflectors position on the patient image, after the acquisition trough the peripheral of image acquisition and coaxial illumination. This instrument doesn't consist only in a program, but in a whole system realized trough or controlled by a software, just like other inventions having obtained industrial protection (only an example: ABS braking system in automobiles- governed by a software). The patent on this invention should logically include the software as well, being itself a comprehensive part of the system.

This kind of protection is by now recognized when, as here described, the discovery produces a technical result 'supplying a technical solution to a technical problem' (Petition's Commission of European Office for patent, T 1173/97). Essentially, this patent's request, concerns not only the software as such but the complete technical process - the diagnostic system of primary and secondary scoliosis or some paramorphism of scoliosis, as well as the other applications described in the Summary, without using radiography. All that realized trough the use of the different equipments described at point 3, carried out under its control (according to the UEB T 26/86 decision). So the use of the software with the use of the other equipments of Spinal Meter, produces a technical effect verifiable because of its belonging to technology's area. It's so necessary to consider the invention in its entirety paying attention to the technical aspect prevalence; invention finalized also trough the software applied to the processor (principle accepted in the decision of UEV T209/91).

This invention has evidently all the requirements needed by the rule in force for the patent obtainment: innovation, industrial applicability, inventiveness with technical meaning not obvious in the present knowledge state. Here again, the function and the role of the software is deserved the protection because of its inclusion in a more sophisticated and complex whole, which is the complete diagnostic system.

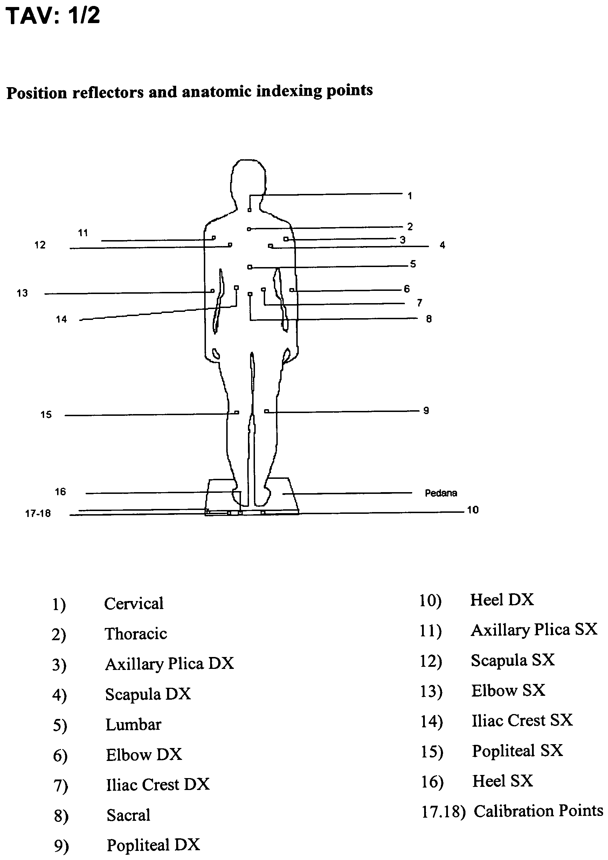

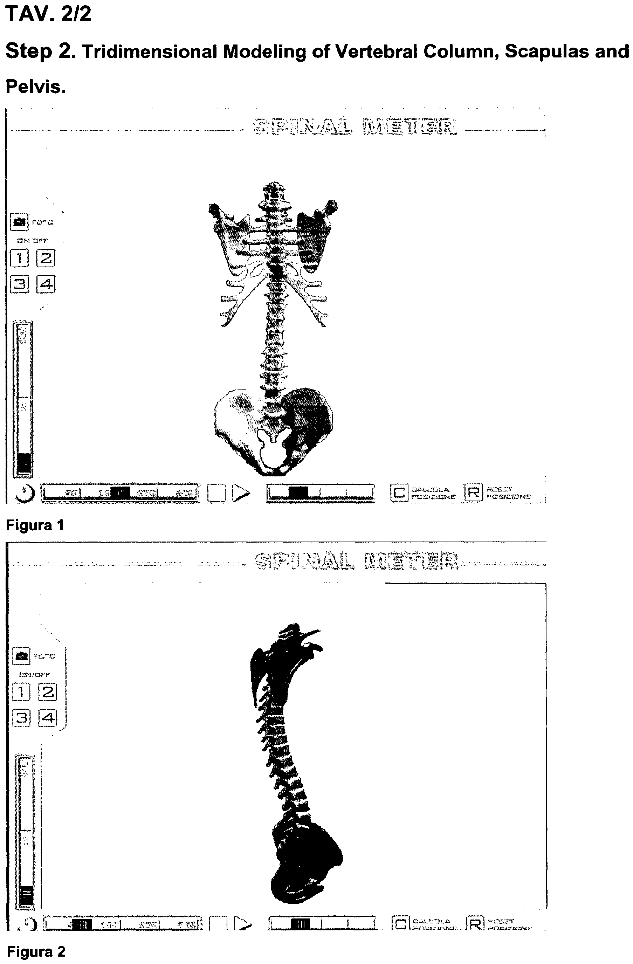

## Claims

1. Device comprising:
- a platform intended to receive a patient,
- position reflectors (1, 2, 3, 4, 5, 6, 7, 8, 9, 11, 12, 13, 14, 15) intended to be applied to the patient body,
- an illumination system suitable to illuminate said position reflectors applied to the patient body,
- a personal computer,
- an image acquisition system connected to said personal computer and suitable to acquire a photographic image of the patient body with said position reflectors, and
- a software stored in said personal computer for acquisition and elaboration of data from said acquisition system, in order to generate image in 3D from said position reflectors on the patient body,
wherein said platform comprises:
- a first position reflector (10) intended to be positioned in correspondence of the right heel of the patient arranged on the platform,
- a second position reflector (16) intended to be positioned in correspondence of the left heel of the patient arranged on the platform, and
- two calibration points (17, 18).

2. Device of claim 1, wherein said position reflectors intended to be applied to the patient body comprise:
- a position reflector (9) intended to be positioned in correspondence of the right popliteal;
- a position reflector (15) intended to be positioned in correspondence of the left popliteal.

3. Device according to claim 1 or 2, wherein said position reflectors intended to be applied to the patient body comprise:
- two position reflector (3, 6) intended to be positioned in correspondence of right axillary plica and right elbow; and
- two position reflector (11, 13) intended to be positioned in correspondence of left axillary plica and left elbow.

4. Device of anyone of the preceding claims, wherein said position reflectors intended to be applied to the patient body comprises:
- four position reflectors (1, 2, 5, 8) intended to be positioned respectively in correspondence of cervical, thoracic, lumbar and sacral,
- two position reflectors (4, 12) intended to be positioned respectively in correspondence of right scapula and left scapula,
- two position reflectors (7, 14) intended to be positioned respectively in correspondence of right iliac crest and left iliac crest.

## Patentansprüche

1. Vorrichtung, umfassend:
eine Plattform, die dafür vorgesehen ist, einen Patienten aufzunehmen,
Positionsreflektoren (1, 2, 3 ,4, 5, 6, 7, 8, 9, 11, 12, 13, 14, 15), die dafür vorgesehen sind, an dem Körper des Patienten angewendet zu werden,
einen Personal Computer,
ein Bildaufnahmesystem, das mit dem Personal Computer verbunden ist und geeignet ist, ein photographisches Bild des Körpers des Patienten mit den Positionsreflektoren aufzunehmen, und
eine Software, die in dem Personal Computer zur Aufnahme und Bearbeitung von Daten von dem Aufnahmesystem vorgesehen ist, um ein Bild in 3D von den Positionsreflektoren an dem Körper des Patienten zu erzeugen, wobei
die Plattform umfasst:
einen ersten Positionsreflektor (10), der dafür vorgesehen ist, entsprechend der rechten Ferse des auf der Plattform angeordneten Patienten positioniert zu werden,
einen zweiten Positionsreflektor (16), der dafür vorgesehen ist, entsprechend der linken Ferse des auf der Plattform angeordneten Patienten positioniert zu werden, und
zwei Eichpunkte (17, 18).

2. Vorrichtung nach Anspruch 1, wobei die Positionsreflektoren, die zur Anwendung an dem Körper des Patienten vorgesehen sind, umfassen:
einen Positionsreflektor (9), der dafür vorgesehen ist, entsprechend der rechten Kniekehle positioniert zu werden;
einen Positionsreflektor (15), der dafür vorgesehen ist, entsprechend der linken Kniekehle positioniert zu werden.

3. Vorrichtung nach Anspruch 1 oder 2, wobei die Positionsreflektoren, die zur Anwendung an dem Körper des Patienten vorgesehen sind, umfassen:
zwei Positionsreflektoren (3, 6), die vorgesehen sind, entsprechend der rechten Plica Axillaris und dem rechten Ellbogen positioniert zu werden; und
zwei Positionsreflektoren (11, 13), die vorgesehen sind, entsprechend der linken Plica Axillaris und dem linken Ellbogen positioniert zu werden.

4. Vorrichtung nach einem der vorhergehenden Ansprüche, wobei die Positionsreflektoren, die zur Anwendung an dem Körper des Patienten vorgesehen sind, umfassen:
vier Positionsreflektoren (1, 2, 5, 8), die vorgesehen sind, jeweils in Entsprechung Hals, Thorax, Lende und Kreuzbein positioniert zu werden,
zwei Positionsreflektoren (4, 12), die vorgesehen sind, jeweils entsprechen rechtem Schulterblatt und linkem Schulterblatt positioniert zu werden,
zwei Positionsreflektoren (7, 14), die vorgesehen sind, jeweils entsprechend rechtem Beckenkamm und linkem Beckenkamm angeordnet zu werden.

## Revendications

1. Dispositif comprenant :
une plateforme prévue pour recevoir un patient,
des réflecteurs de position (1, 2, 3, 4, 5, 6, 7, 8, 9, 11, 12, 13, 14, 15) prévus pour être appliqués sur le corps du patient,
un système d'éclairage approprié pour éclairer desdits réflecteurs de position appliqués sur le corps du patient,
un ordinateur personnel,
un système d'acquisition d'image raccordé audit ordinateur personnel et approprié pour acquérir une image photographique du corps du patient avec lesdits réflecteurs de position, et
un logiciel stocké dans ledit ordinateur personnel pour l'acquisition et l'élaboration de données dudit système d'acquisition afin de générer l'image en 3D à partir desdits réflecteurs de position sur le corps du patient,
dans lequel ladite plateforme comprend :
un premier réflecteur de position (10) prévu pour être positionné en correspondance du talon droit du patient disposé sur la plateforme,
un second réflecteur de position (16) prévu pour être positionné en correspondance du talon gauche du patient disposé sur la plateforme, et
deux points de calibrage (17, 18).

2. Dispositif selon la revendication 1, dans lequel lesdits réflecteurs de position prévus pour être appliqués sur le corps du patient comprennent :
un réflecteur de position (9) prévu pour être positionné en correspondance du poplité droit ;
un réflecteur de position (15) prévu pour être positionné en correspondance du poplité gauche.

3. Dispositif selon la revendication 1 ou 2, dans lequel lesdits réflecteurs de position prévus pour être appliqués sur le corps du patient comprennent :
deux réflecteurs de position (3, 6) prévus pour être positionnés en correspondance de la plicature axillaire droite et du coude droit ; et
deux réflecteurs de position (11, 13) prévus pour être positionnés en correspondance de la plicature axillaire gauche et du coude gauche.

4. Dispositif selon l'une quelconque des revendications précédentes, dans lequel lesdits réflecteurs de position prévus pour être appliqués sur le corps du patient comprennent :
quatre réflecteurs de position (1, 2, 5, 8) prévus pour être positionnés respectivement en correspondance des positions cervicales, thoraciques, lombaires et sacrales,
deux réflecteurs de position (4, 12) prévus pour être positionnés respectivement en correspondance de l'omoplate droite et de l'omoplate gauche,
deux réflecteurs de position (7, 14) prévus pour être positionnés respectivement en correspondance de la crête iliaque droite et de la crête iliaque gauche.
